# EUROPEAN PATENT APPLICATION

(11) **EP 1 739 742 A1**
(43) Date of publication of application: **03.01.2007**
(21) Application number: 06253252.8
(22) Date of filing: 22.06.2006
(51) Int. Cl.: H01L 23/367, H01L 23/373

(54) **Thermally conductive member and cooling system using the same**

(30) Priority: 30.06.2005 JP 2005192437
(71) Applicant: POLYMATECH CO., LTD., Tokyo 103-8424 (JP); Otsuka Electric Co., Ltd., Nakahara-ku, Kawasaki-shi Kanagawa-ken 211-0053 (JP)
(72) Inventor: Yamazaki, Jun, c/o Polymatech Co., Ltd., Kita-ku Tokyo 114-0014 (JP); Ohta, Mitsuru, c/o Polymatech Co., Ltd., Kita-ku Tokyo 114-0014 (JP); Ozawa, Motoki, c/o Polymatech Co., Ltd., Kita-ku Tokyo 114-0014 (JP); Fujiwara, Kikuo, c/o Otsuka Electric Co., Ltd., Kawasaki-shi Kanagawa-ken 211-0053 (JP)
(74) Representative: Sherrard-Smith, Hugh

(57) **Abstract**

A thermally conductive member (100) including a thermal diffusion sheet (10) having at least one opening (20) with an inner periphery; and a thermally conductive elastomer piece (30) passing through the opening (20) of the sheet (10) is provided. The thermally conductive elastomer piece (30) includes at least one base portion (30c) fitting with the inner periphery of the opening (20) of the thermal diffusion sheet (10) and at least one projecting portion (30a, 30b) connected to the base portion (30c) and projecting out from the surface of the thermal diffusion sheet (10).

## Description

The present invention relates to a thermally conductive member including a thermal diffusion sheet, and to a cooling system using the thermally conductive member.

Recently, electronic equipment such as notebook type personal computers, mobile phones, and digital still cameras are progressively being reduced in weight, thickness, and size. At the same time, this electronic equipment is achieving more sophisticated functions, larger information storage capacities and higher information processing speeds. Along with those developments, problems caused by heat generated from precision electronic parts such as ICs and CPUs incorporated in the electronic equipment are becoming serious.

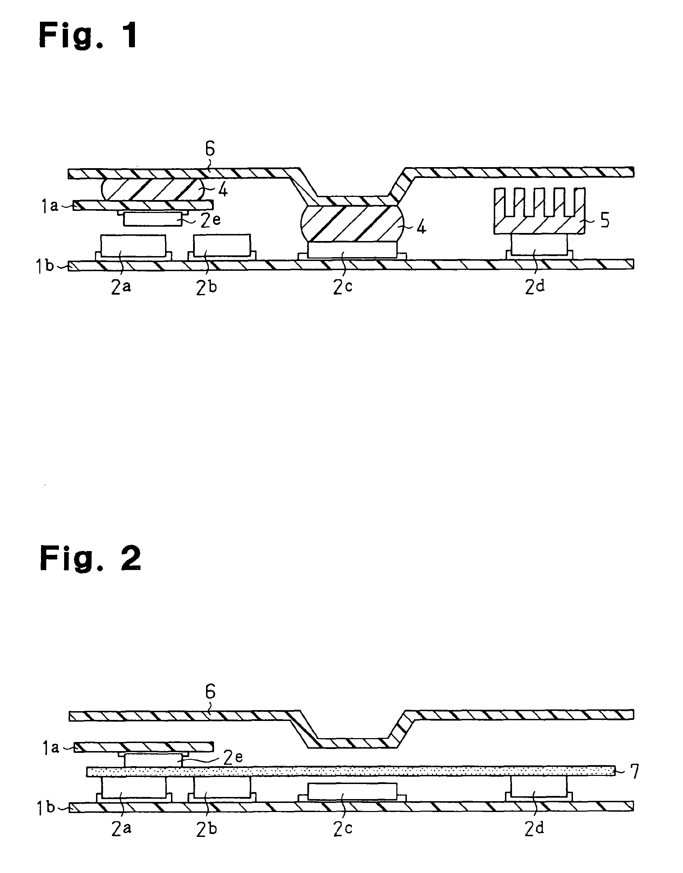

A first problem caused by such heat includes failure or malfunction of precision electronic parts that can be a heat source in the electronic equipment due to a rise in temperature of these precision electric parts, or an adverse influence on the parts other than the heat source, which is exerted due to a rise in internal temperature of the electronic equipment caused by the heat generated from the heat source. To cope with this problem, a countermeasure shown in Fig. 1 has conventionally been taken, for example.

Referring to Fig. 1, electronic parts 2a, 2b, 2c, and 2d, which are heat sources, are mounted on a top surface of a substrate 1b in an electronic apparatus. An electronic part 2e is mounted on the underside of a substrate 1a. In this example, a heat sink 5 is mounted on the electronic part 2d to radiate the heat from the electronic part 2d to an inner space of the electronic equipment. Further, each of two thermally conductive members 4 is interposed between the electronic part 2c and a housing 6 of the electronic equipment and between a portion of the top surface of the substrate 1a opposing the electronic part 2e of the same and the housing 6. These thermally conductive members 4 thermally connect the respective electronic parts 2c and 2e as heat sources to the housing 6 serving as a cooling member so as to radiate the heat from the electronic parts 2c and 2e and the heat from the inside of the electronic equipment to an outside of the electronic equipment through the housing 6. A flexible thermally conductive sheet is often used as such a thermally conductive member in order to absorb irregularities and accumulated dimensional tolerance of the heat sources and an expansion of each part by the heat. This method provides a heat radiation effect to some extent. However, this method cannot be applied to some parts, such as the electronic parts 2a and 2b shown in Fig. 1, that have no space around them to mount a heat sink thereon for dispersing heat or thermally conductive members for thermally connecting those electronic parts to the housing. In case of the electronic part 2e that is connected to the housing 6 by the thermally conductive member 4 through the substrate 1a, the heat radiation effect from the thermally conductive members 4 becomes very low. Therefore, the heat builds up at a site where it is difficult to deal with the heat either at all or satisfactorily and causes reduction in lifetime or performance such as arithmetic processing of the electronic parts. Further, installation of the heat radiation sheet or the heat sink for respective heat sources requires a large number of steps in the manufacturing process, thereby greatly reducing productivity of the parts.

A second problem caused by the heat resides in that the heat generated from the electronic equipment such as notebook type personal computers, mobile phones, or digital still cameras, which may come into contact with users can cause an unpleasant or uncomfortable feeling for users. Fig. 2 is a sectional view of a typical solution to this second problem. According to this example, in the electronic equipment, a thermal diffusion sheet 7 is opposed to the electronic parts 2a, 2b, 2c and 2d mounted on the top surface of the substrate 1b and to the electronic part 2e mounted on the underside of the substrate 1a. This structure allows the heat generated from the electronic parts 2a-2e producing the heat to be conducted to the thermal diffusion sheet 7. As a result, the heat is evenly diffused along the surfaces of the sheet 7, thereby preventing the temperature around each of the electronic parts from locally rising. A metal sheet having a relatively high thermal conductivity such as magnesium, aluminum, and copper sheets may be used as the thermal diffusion sheet 7. However, for the purpose above-mentioned, a graphite sheet having a higher thermal conductivity in a direction parallel to the surface of the sheet is particularly preferred as the thermal diffusion sheet 7. For example, Japanese Laid-Open Patent Publication No. 2001-287299 discloses a thermally conductive sheet including a graphite sheet and a thin insulating film layer provided on a surface of the graphite sheet. However, in general, the graphite sheet has lower flexibility in comparison with a heat radiation sheet made of a polymer material. Accordingly, a contact resistance between the graphite sheet and the surface of the heat source cannot be reduced enough. If an excessive load is applied to an electronic part so as to reduce the contact resistance, a failure of the electronic part may occur. A graphite sheet having low flexibility cannot compensate for variations in height of electronic parts being heat sources. For example, space is left between the graphite sheet and an electronic part having a smaller height than other electronic parts, like the electronic part 2c as shown in Fig. 2. As a result, the heat generated from the electronic parts may not be conducted to the graphite sheet efficiently. To cope with this problem, Japanese Laid-Open Patent Publication No. 2004-243650 discloses a thermally conductive sheet having a silicone elastomer layer on one side or both sides of a graphite sheet layer through an adhesive layer to improve its adhesion to parts to be mounted.

However, when this graphite sheet is used as the thermal diffusion sheet, as shown in Fig. 2, the heat generated from the electronic parts 2a-2e is merely diffused into the inside of the thermal diffusion sheet and then an inner space of the equipment through the thermal diffusion sheet. Therefore, when the inner space of the electronic equipment is saturated with the heat, there is no escape of the heat. As a result, when the electronic equipment is used for a long time, the temperature of the equipment entirely rises, which may lead to such inconvenience as a failure or malfunctioning of the electronic equipment or a reduction in its operation speed. The thermal conductivity in the thickness direction of the graphite sheet is lower than the thermal conductivity in direction parallels to the surface of the graphite sheet.

In the thermally conductive sheet, as disclosed in Japanese Laid-Open Patent Publication No.2004-243650, in which the elastomer layer is formed on the surface of the graphite sheet, the direction of the heat conduction from the elastomer layer to the graphite sheet corresponds to the thickness direction of the graphite sheet. Therefore, the heat from the elastomer layer does not efficiently conduct to the graphite sheet. Accordingly, the graphite sheet cannot absorb the heat from the heat source through the elastomer layer sufficiently.

A forced cooling system including a cooling fan or Peltier element also may be used. However, use of such a cooling system may results in the larger size of the electronic equipment, the higher power consumption, and the increased cost for the equipment.

It is therefore an object of the present invention to provide a thermally conductive member which can prevent local generation of heat from a heat source such as an electronic part in electronic equipment, and which can effectively conduct the heat to a cooling member such as a housing of the equipment to successfully radiate the heat to the outside of the electronic equipment. The present invention also provides a cooling system using the thermally conductive member above-mentioned.

In one aspect, the present invention provides a thermally conductive member including a thermal diffusion sheet having at least one opening with an periphery and a thermally conductive elastomer piece passing through the opening of the sheet. The thermally conductive elastomer piece includes at least one base portion fitting with the inner periphery of the opening of the thermal diffusion sheet and at least one projecting portion connected to the base portion and projecting out from the surface of the thermal diffusion sheet.

In another aspect, the present invention provides a cooling system including a thermal diffusion sheet having at least one opening with an inner periphery and a thermally conductive elastomer piece passing through the opening of the sheet. The thermally conductive elastomer piece includes at least one base portion fitting with an inner periphery of the opening of the thermal diffusion sheet and at least one projecting portion connected to the base portion and projecting out from the surface of the thermal diffusion sheet. The cooling system further includes a cooling member in close contact with the projecting portion of the thermally conductive elastomer piece.

The present invention also provides a method for producing the thermally conductive member above-mentioned. In one aspect, the method includes forming the at least one opening through the thermal diffusion sheet; preparing a composition containing a polymer matrix and a thermally conductive filler; and insert molding the composition with the thermal diffusion sheet to produce the thermally conductive elastomer piece passing through the opening of the thermal diffusion sheet.

In another aspect, the method includes forming the at least one opening through the thermal diffusion sheet; preparing a composition containing a polymer matrix and a thermally conductive filler; molding the composition into the thermally conductive elastomer piece in a predetermined shape; and assembling the thermally conductive elastomer piece with the thermal diffusion sheet through the opening of the sheet.

Other aspects and advantages of the invention will become apparent from the following description, taken in conjunction with the accompanying drawings, illustrating by way of example the principles of the invention.

The invention, together with objects and advantages thereof, may best be understood by reference to the following description of the presently preferred embodiments together with the accompanying drawings in which:
Fig. 1 is a diagram showing a conventional countermeasure for heat generation;
Fig. 2 is a diagram showing another conventional countermeasure for heat generation;
Fig. 3 is a perspective view of a thermally conductive member according to an embodiment of the present invention;
Fig. 4 is a sectional view of the thermally conductive member according to the embodiment of the present invention;
Fig. 5 is a perspective view of a modification of the thermally conductive member;
Fig. 6 is a perspective view of another modification of the thermally conductive member;
Fig. 7 is a perspective view of still another modification of the thermally conductive member; and
Fig. 8 is a sectional view of a cooling system of the present invention.

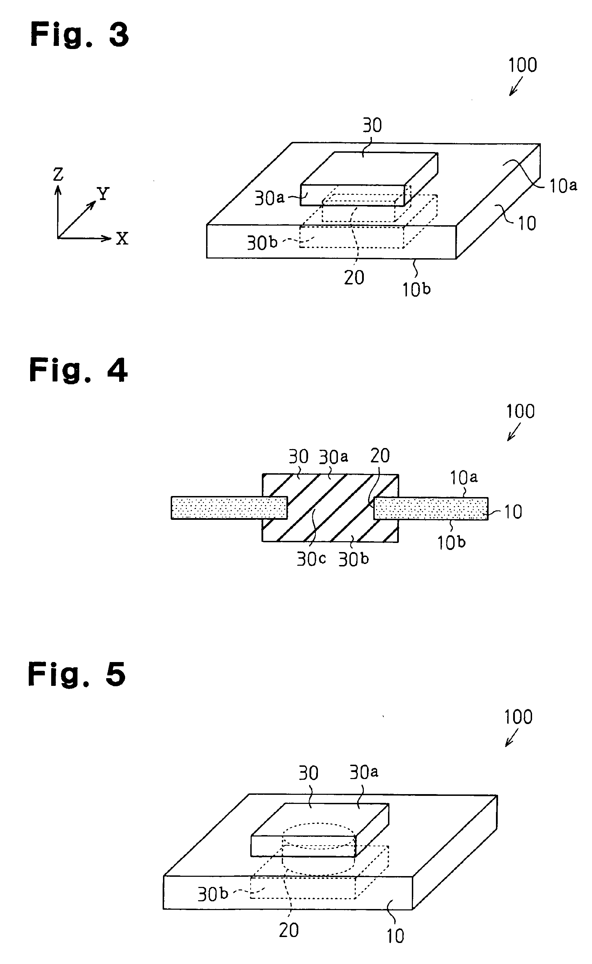

With reference to Figs.3, 4, and 8, a preferred embodiment of the present invention will be described hereafter

A thermally conductive member 100 shown in Fig. 3 includes a thermal diffusion sheet 10 having an opening 20 and a thermally conductive elastomer piece 30 passing through the opening 20 of the thermal diffusion sheet 10.

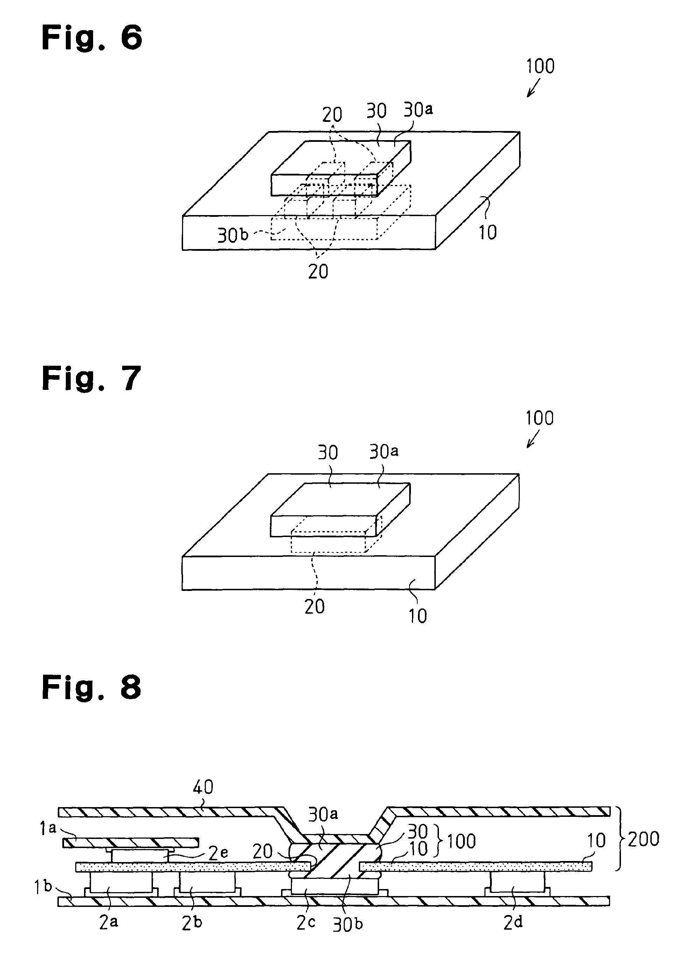

As shown in Fig. 8, the thermally conductive elastomer piece 30 is arranged between in close contact with an electronic part 2c that is a heat source and a cooling member 40 such as a housing for equipment. The thermally conductive elastomer piece 30 conducts the heat from the heat source to the thermal diffusion sheet 10 and to the cooling member 40 as well. When the thermally conductive elastomer piece 30 is placed into contact with the respective heat source and the cooling member as shown Fig.8, the thermally conductive elastomer piece 30 may be elastically deformed by an application of load. As a result, the close contact between the thermally conductive elastomer piece 30 and the respective heat source and the cooling member can be ensured.

The thermally conductive sheet 10 has a function for diffusing the heat conducted from the thermally conductive elastomer piece 30 within in the sheet in a direction parallel to the surface of the sheet. Therefore, it is preferable that the thermal conductivity of the thermal diffusion sheet 10 in a direction parallel to the surface of the sheet is higher than the thermal conductivity in the thickness direction of the sheet.

As shown in Fig. 4, the thermally conductive elastomer piece 30 of this embodiment has a base portion 30c fitting with the inner periphery of the opening 20 of the thermal diffusion sheet 10 and a pair of projecting portions 30a and 30b connected to the base portion 30c and projecting out from each plane of the top surface 10a and the under surface 10b of the thermal diffusion sheet 10 respectively. The projecting portions 30a and 30b of the thermally conductive elastomer piece 30 also project peripherally outward from the edges of the opening 20 and extend over the top surface 10a and the under surface 10b of the thermal diffusion sheet 10, respectively. Therefore, each cross-sectional area of the projecting portions 30a and 30b is larger than that of the opening 20. The sizes and shapes of the opening 20 and the base portion 30c corresponding thereto can be changed in consideration of the shape and surface area of the heat source and the strength of the thermal diffusion sheet 10.

In the embodiment shown in Fig. 3, the cross-sectional shapes of the opening 20 and the base portion 30c are rectangular. However, the opening 20 and the base portion 30c may have any cross-sectional shape. For example, they may have circular cross-sections as shown in Fig. 5. The number of the openings 20 and the number of the base portions 30c are not particularly limited. As shown in Fig. 6, the thermal diffusion sheet 10 may have a plurality of openings 20. In this case, the thermally conductive elastomer piece 30 has a plurality of base portions 30c corresponding to the number of the openings 20. The projecting portions 30a and 30b of the thermally conductive elastomer piece 30 are desirably designed in consideration of their contact areas with the heat source and the cooling member, such as a heat sink or housing, for radiating the heat to the outside environment. It is particularly preferable that the projecting portions 30a and 30b have larger surface areas than the contact areas with the heat source and the cooling member.

The thermally conductive member 100 in this embodiment is arranged in the electronic equipment in such a manner that the projecting portion 30b of the thermally conductive elastomer piece 30 is in close contact with a heat source such as an electronic part and the projecting portion 30a of the thermally conductive elastomer piece 30 is in close contact with a cooling member such as the housing of the electronic equipment.

In the thermally conductive member 100 of Fig. 3, the thermally conductive elastomer piece 30 is incorporated with the thermal diffusion sheet 10 by passing through the opening 20, such that a part of the thermally conductive elastomer piece 30 contacts the inner peripheral surface defining the opening 20 of the thermal diffusion sheet 10. Therefore, the heat transmitted from the heat source to the thermally conductive elastomer piece 30 is conducted to the thermal diffusion sheet 10 through the inner peripheral surface of the opening 20 of the thermal diffusion sheet 10 and further rapidly diffused into the sheet 10. This can prevent generation of a portion having a locally raised temperature, so-called "heat spot", at a position opposed to the heat source.

Since the conduction of the heat from the thermally conductive elastomer piece 30 shown in Fig. 3 to the thermal diffusion sheet 10 is carried out through the inner peripheral surface of the opening 20 as described above, the direction of this heat conduction corresponds to a direction parallel to the surface of the thermal diffusion sheet 10 (for example, in an X or Y direction). Therefore, when a thermal diffusion sheet having thermal conductivity in a direction parallel to the surface that is higher than the thermal conductivity in the thickness direction is used as the thermal diffusion sheet 10, the conduction of the heat from the thermally conductive elastomer piece 30 to the thermal diffusion sheet 10 can be carried out more efficiently.

In the thermally conductive member 100, the thermally conductive elastomer piece 30 extends through the thermal diffusion sheet 10 in the thickness direction (in the Z direction) of the sheet. This structure allows the thermally conductive elastomer piece 30 to efficiently absorb the heat from the heat source in contact with the projecting portion 30b of the thermally conductive elastomer piece 30 to transmit the absorbed heat to the thermal diffusion sheet 10 as described above. In addition, the thermally conductive elastomer piece 30 can further conduct the absorbed heat to the cooling member disposed adjacent to the thermally conductive member 100 through the projecting portion 30a.

As described above, the thermally conductive member 100 of this embodiment can diffuse the heat from the heat source effectively and can conduct the heat to the cooling member effectively at the same time. In this case, a part of the heat from the heat source is diffused rapidly and evenly along the thermal diffusion sheet 10 as described above. Therefore, when the housing of the electric equipment is used as the cooling member, a heat spot is hardly produced on the housing by the heat conducted to the housing from the heat source through the thermally conductive elastomer piece 30.

When the thermally conductive elastomer piece 30 is placed into contact with the heat source and the cooling member, the thermally conductive elastomer piece 30 may be elastically deformed by application of a load thereto. As a result, close contact between the thermally conductive elastomer piece 30 and each of the heat source and the cooling member can be ensured.

The thermally conductive elastomer piece 30 may have projecting portions 30a and 30b having a larger cross-sectional area than the cross-sectional area of the opening 20 on both sides of the thermal diffusion sheet 10 as shown in Figs. 3 to 7. This structure can prevents the thermally conductive elastomer piece 30 from falling off from the thermal diffusion sheet 10.

As shown in Fig. 7, the projecting portion 30b of the thermally conductive elastomer piece 30 may be omitted. In this case, the bottom surface of the thermally conductive elastomer piece 30 situated in the opening 20 is to be in contact with the heat source. According to this structure, the thermally conductive member 100 can be arranged in a narrow mounting space in the electronic equipment in addition to the above-mentioned effect of the embodiment.

Fig. 8 shows a cooling system 200 including the thermally conductive member 100 shown in Figs. 3 and 4. This cooling system 200 is mounted to substrates 1a and 1b having electronic parts 2a, 2b, 2c, 2d and 2d. The cooling system 200 is composed of the thermally conductive member 100 shown in Fig. 3 and a cooling member 40. More specifically, the cooling system 200 includes the thermal diffusion sheet 10 having the opening 20, the thermally conductive elastomer piece 30 passing through the opening 20, and the cooling member 40 in contact with the thermally conductive elastomer piece 30. The cooling member 40 is provided in contact with the top surface of the projecting portion 30a of the thermally conductive elastomer piece 30. The cooling member 40 is a member serving to radiate the heat from the thermally conductive elastomer piece 30 to the outside environment. In the cooling system 200 of Fig. 8, the cooling member 40 is the housing of electronic equipment in which the cooling system 200 is mounted. However, the cooling member 40 may be a conventional cooling device such as a heat sink. In this embodiment, the cooling system 200 is disposed opposing to the substrates 1a and 1b so that the bottom surface of the projecting portion 30b of the thermally conductive elastomer piece 30 closely contacts the top surface of the electronic part 2c generating a large amount of the heat such as a semiconductor device, while the thermal diffusion sheet 10 contacts other electronic parts 2a, 2b, 2d, and 2e. The heat generated from the electronic part 2c is diffused into the thermal diffusion sheet 10 through the thermally conductive elastomer piece 30, transmitted to the cooling member 40 from the thermally conductive elastomer piece 30, and further radiated to the outside environment from the cooling member 40. The heat generated from the electronic parts 2a, 2b, 2d and 2e can be directly transmitted to the thermal diffusion sheet 10 to be diffused into the sheet 10 and can also be transmitted from the thermal diffusion sheet 10 to the cooling member 40 through the thermally conductive elastomer piece 30 and be radiated to the outside environment therefrom.

The cooling system 200 of the above-mentioned embodiment has the following effect in addition to the effect obtained by the thermally conductive member 100.

In the cooling system 200 above-mentioned, the thermally conductive elastomer piece 30 is incorporated with the thermal diffusion sheet 10 by passing through the opening 20 of the sheet 10 in the thickness direction of the sheet and is in close contact with the cooling member 40. Owing to this structure, the thermally conductive elastomer piece 30 can efficiently absorb the heat from the heat source in contact with the projecting portion 30b and transmits the heat to the thermal diffusion sheet 10 for diffusing the heat along the sheet as described above, and also can effectively conduct the heat to the cooling member through the projecting portion 30a to radiate the heat to the outside environment from the cooling member efficiently.

Each structural element of the thermally conductive member of the present invention will be described in detail hereinbelow.

### <Thermal diffusion sheet>

The thermal diffusion sheet 10 diffuses the heat in a direction parallel to the surface of the sheet 10 and further radiates the heat to the outside environment from the periphery and the surface of the sheet. To ensure this heat diffusion function, the thermal diffusion sheet 10 must have a thermal conductivity of 100 W/m·K or more, preferably 150 to 900 W/m·K in a direction parallel to the surface of the sheet. Preferably, the thermal diffusion sheet 10 has a higher thermal conductivity in a direction parallel to the surface than the thermal conductivity in the thickness direction of the sheet.

Although a metal sheet made of a metal alone such as copper or aluminum has a relatively high thermal conductivity (copper: about 400 W/m·K, aluminum: 180 to 200 W/m·K) in general, such a metal sheet has isotropic thermal conductivity. Such a metal sheet when used as a thermal diffusion sheet diffuses the heat generated from, for example, the electronic parts 2a, 2b and 2d shown in Fig. 8 in a direction parallel to the surface, and also efficiently transmits the heat in the thickness direction at portions opposed to these heat sources. As a result, the temperature of each of the portions opposed to these heat sources rises locally, thereby producing hot spots.

In contrast to this, a graphite sheet generally has an extremely higher thermal conductivity (100 to 800 W/m·K) in a direction parallel to the surface as compared with that in the thickness direction. Therefore, the graphite sheet can transmit and diffuse the heat rapidly in a direction parallel to the surface rather than in the thickness direction of the sheet. Consequently, the graphite sheet is particularly preferred as the thermal diffusion sheet 10 used in the thermally conductive member of the present invention.

It is effective to use a composite sheet consisting of a graphite sheet having a higher thermal conductivity in a direction parallel to the surface, and a metal layer such as an aluminum foil having excellent isotropic thermal conduction properties as the thermal diffusion sheet 10. In this case, the aluminum foil may be provided on only one side of the graphite sheet or on both sides of the graphite sheet. The aluminum foil may be provided on a part of the surface of the graphite sheet or over the entire surface of the graphite sheet. Other metal foils made of, for example, copper, copper alloy, and gold, may also be used as the metal layer of the composite sheet. When such the composite sheet is used as the thermal diffusion sheet 10, the aluminum foil provided on the surface of the thermal diffusion sheet 10 efficiently conducts the heat from the thermally conductive elastomer piece 30 to the graphite sheet residing inside of the thermal diffusion sheet 10. The aluminum foil can also radiate the heat diffused into the graphite sheet to the outside environment from the surface of the graphite sheet. Such the composite sheet consisting of a graphite sheet and an aluminum foil has improved mechanical strength and shape retainability as compared with a sheet made of graphite alone.

The graphite sheet has a much higher thermal conductivity in a direction parallel to the surface than thermal conductivity in the thickness direction as described above. Meanwhile, since the heat conduction from the thermally conductive elastomer piece 30 to the thermal diffusion sheet 10 is carried out through the inner peripheral surface of the opening 20, the direction of this heat conduction corresponds to a direction parallel to the surface of the thermal diffusion sheet 10. Therefore, when the graphite sheet or the composite sheet consisting of a graphite sheet and an aluminum foil is used as the thermal diffusion sheet 10, the heat conduction from the thermally conductive elastomer piece 30 to the thermal diffusion sheet 10 is extremely efficient as compared with when it is carried out through the surface of the thermal diffusion sheet 10.

The thickness of the thermal diffusion sheet 10, which is not particularly limited, is preferably 10 to 550 µm in consideration of installation in limited mounting space for electronic equipment. When the thermal diffusion sheet 10 is thinner than 5 µm, it may become fragile, easily breaks, and disadvantageously has a small heat capacity. When the thickness of the thermal diffusion sheet 10 is larger than 550 µm, the stiffness of the sheet becomes higher, thereby reducing work efficiency. Also, such a thicker thermal diffusion sheet is not economically preferred.

### <Thermally conductive elastomer>

The thermally conductive elastomer piece 30 is made of a composition including a polymer matrix material and a thermally conductive filler.

The thermally conductive elastomer piece 30 may have isotropic or anisotropic thermal conduction properties. When the thermally conductive elastomer piece 30 has anisotropic thermal conduction properties, the thermal conductivity in the direction substantially perpendicular to the surface of the thermal diffusion sheet 10 (for example, in the Z direction of Fig. 3) is preferably set higher than the thermal conductivity in a direction parallel to the surface (for example, in the X or Y direction of Fig. 3). Thereby, the thermally conductive elastomer piece 30 can absorb the heat from the heat source in contact with the projecting portion 30b effectively, transmit the heat to the thermal diffusion sheet 10 rapidly and further efficiently conduct the heat to the cooling member in contact with the projecting portion 30a through the projecting portion 30a.

When the hardness of the thermally conductive elastomer is 50 or less measured by a type A durometer in accordance with Japanese Industrial Standard ("JIS") K6253 (corresponding to ISO 7619-1), the thermally conductive elastomer advantageously has excellent conformity to the contact surfaces of the heat source and the cooling member.

The thermally conductive elastomer piece 30 may have electric insulating properties. This is particularly effective when there is a possibility that an electric failure with a heat source may occur, for example, when the cooling member is electrically conductive.

### <Thermally conductive filler in thermally conductive elastomer>

The thermally conductive filler contained in the thermally conductive elastomer piece 30 is preferably at least one selected from carbon fibers, carbon nanotubes, metal nitrides, metal oxides, metal carbides, and metal hydroxides.

The thermally conductive filler above-mentioned may have isotropic thermal conduction properties or may have anisotropic thermal conduction properties. When the thermally conductive filler has anisotropic thermal conduction properties, the thermally conductive filler is oriented in a specific direction in the thermally conductive elastomer to improve the thermal conductivity in the specific direction of the obtained thermally conductive elastomer. For example, carbon fibers have a high thermal conductivity in the axial direction rather than the diameter direction of the fibers. Such carbon fibers are oriented such that the axial direction of the each fiber is aligned substantially parallel to the Z direction of Fig. 3 in the thermally conductive elastomer, namely the direction substantially perpendicular to the surface of the thermal diffusion sheet 10. Thereby, the thermal conductivity of the thermally conductive elastomer piece 30 in the direction substantially perpendicular to the surface of the thermal diffusion sheet 10 can be easily set higher than the thermal conductivity in the direction parallel to the surface of the thermal diffusion sheet 10.

Examples of the method for orienting the thermally conductive filler in the thermally conductive elastomer include methods employing a flow field, a shear field, a magnetic field, and an electric field. Particularly, when the thermally conductive filler is at least one selected from carbon fibers, carbon nanotubes, metal nitrides, metal oxides, metal carbides, and metal hydroxides, the thermally conductive filler is preferably oriented by a magnetic field using a magnetic anisotropy that is specific to each of these thermally conductive fillers. In this case, thermally conductive polymer composition containing the thermally conductive filler above-mentioned is externally applied with a magnetic field to orient the thermally conductive filler in a direction substantially parallel or perpendicular to the lines of magnetic force. This method can provide the efficient orientation of the thermally conductive filler and easy control of the orientation direction to any desired directions.

The fiber diameter of the carbon fibers is preferably 5 to 20 µm, more preferably 5 to 15 µm, and particularly preferably 8 to 12 µm. If the fiber diameter is smaller than 5 µm or larger than 20 µm, the productivity of the carbon fibers may be disadvantageously decreased. The average length of the carbon fibers is preferably 5 to 500 µm, more preferably 15 to 100 µm, and particularly preferably 15 to 45 µm. If the average length is shorter than 5 µm, contacts between carbon fibers in the thermally conductive elastomer piece 30 for establishing heat conduction routes decrease, thereby reducing the thermal conductivity of the thermally conductive elastomer piece 30. When the average length is longer than 500 µm, the carbon fibers become too bulky to be compound into a polymer matrix material in a high concentration. The average length value of the carbon fibers can be calculated from a particle size distribution measured by a laser diffraction system.

The carbon fibers may have surfaces modified by oxidation such as electrolytic oxidation or treatment with a coupling agent or sizing agent. This surface modification can improve wettability or peel strength at the interface with the polymer matrix material, or increase an amount of the fillers that can be mixed into the polymer matrix. Carbon fibers whose surfaces are coated with a metal or ceramics may also be used. Such coating may be accomplished by physical deposition or chemical deposition such as electroless plating, electrolytic plating, vacuum deposition, sputtering, ion plating, coating, immersion or mechano-chemical process for mechanical fixing of fine particles.

Examples of the metal nitride include silicon nitride and aluminum nitride. Examples of the metal oxides include aluminum oxide, silicon oxide, zinc oxide, and magnesium oxide. An example of the metal carbides includes silicon carbide. Examples of the metal hydroxides include aluminum hydroxide and magnesium hydroxide. These metal nitrides, metal oxides, metal carbides, and metal hydroxides to be used as the thermally conductive filler can provide the resultant thermally conductive elastomer with electrically insulative properties.

The amount of the thermally conductive filler in the thermally conductive elastomer is at least 30 vol% or more, particularly preferably 30 to 55 vol%. By mixing the amount of the thermally conductive filler in the above range, the thermal conductivity of the thermally conductive elastomer can be improved without impairing the flexibility of the thermally conductive elastomer.

### <Polymer matrix material in the thermally conductive elastomer>

The polymer matrix material in the thermally conductive elastomer piece 30 is not particularly limited and can be suitably selected according to characteristic properties required for the obtained thermally conductive elastomer such as heat resistance, chemical resistance, productivity, electric insulating properties, and flexibility. Examples of the polymer matrix material include thermoplastic elastomers and crosslinked rubbers.

Examples of the thermoplastic elastomer include styrene-butadiene copolymer, styrene-isoprene block copolymer, hydrogenated products thereof, styrene-based thermoplastic elastomers, olefin-based thermoplastic elastomers, vinyl chloride-based thermoplastic elastomers, polyester-based thermoplastic elastomers, polyurethane-based thermoplastic elastomers, and polyamide-based thermoplastic elastomers.

Examples of the crosslinked rubbers include natural rubber, butadiene rubber, isoprene rubber, styrene-butadiene copolymer rubber, nitrile rubber, hydrogenated nitrile rubber, chloroprene rubber, ethylene-propylene copolymer rubber, chlorinated polyethylene, chlorosulfonated polyethylene, butyl rubber, halogenated butyl rubber, fluorine rubber, urethane rubber, and silicone rubber.

The method of manufacturing the thermally conductive member of the present invention may include steps of: preparing a composition including a polymer matrix material and a thermally conductive filler; and insert molding the composition with the thermal diffusion sheet 10 having the opening 20 to integrate the thermally conductive elastomer with the thermal diffusion sheet 10. Alternatively, a thermally conductive elastomer piece 30 having a predetermined shape may be individually formed from the composition above-mentioned. The obtained thermally conductive elastomer piece 30 may be assembled with the thermal diffusion sheet 10 having the opening 20 to produce the thermally conductive member.

The thermally conductive member of the present invention can prevent the local heat generation in electronic equipment due to heat from a heat source such as an electronic part and can effectively conduct the heat to a cooling member such as a housing of the equipment. Further, the cooling system of the present invention can prevent the local heat generation in electronic equipment due to heat from a heat source such as an electronic part and efficiently radiate the such heat to the outside environment of the electronic equipment.

It should be apparent to those skilled in the art that the present invention can be embodied in many other specific forms without departing from the spirit or scope of the invention. Particularly, it should be understood that the invention may be embodied in the following forms.

Each of the cross-sectional areas of the projecting portions 30a and 30b of the thermally conductive elastomer piece 30 may be the same as the area of the opening 20 of the thermal diffusion sheet 10.

Specific examples of the thermally conductive member of the present invention and the cooling system including the same of the present invention are described below.

### (Example 1)

Firstly, 70 parts by weight of graphitized carbon fibers (manufactured by Nippon Graphite Fiber Corporation, average fiber diameter: 9µm, average fiber length: 100 µm) and 150 parts by weight of aluminum oxide powders (manufactured by Showa Denko K.K., in spherical shape, average particle size: 3.5 µm) as thermally conductive fillers were mixed with 100 parts by weight of liquid silicone rubber (manufactured by GE Toshiba Silicone Co., Ltd.) as a polymer matrix material. The resultant mixture was vacuum defoamed to prepare a thermally conductive polymer composition.

A thermal diffusion sheet 10 consisting of a graphite sheet (manufactured by GrafTech International Ltd.) having a thickness of 0.13 mm, a length of 30 mm, and a width of 60 mm was provided. The thermal conductivity of the graphite sheet in the thickness direction was 7 W/m·K, and thermal conductivity of the graphite sheet in the direction parallel to the surface of the sheet was 240 W/m·K. An opening 20 being 6 mm long and 6 mm wide was formed through the thermal diffusion sheet 10 at a desired position where the thermally conductive elastomer piece 30 will be incorporated thereto. This thermal diffusion sheet 10 was placed in a cavity of a mold in such a manner that the opening 20 is located completely inside of the cavity. Then, said thermally conductive polymer composition was injected into the cavity of the mold and thermally cured. As a result, a thermally conductive member 100 shown in Fig. 3 in which a plate-like thermally conductive elastomer piece 30 (hardness of 40) integrated with the thermal diffusion sheet 10 so as to cover the opening 20 was obtained. The thermally conductive elastomer piece 30 had a thickness of 0.4 mm in total of the portions 30a, 30b, and 30c, and a length of 10 mm and a width of 10 mm in the portion of the projecting portions 30a, 30b.

As for the obtained thermally conductive member 100, the thermal conductivities of the thermally conductive elastomer piece 30 in the directions perpendicular to the surface of the thermal diffusion sheet 10 and parallel to said surface were measured. Both of said thermal conductivities were 3.8 W/m·K.

The top surface of the projecting portion 30a of the thermally conductive elastomer piece 30 of the obtained thermally conductive member 100 was closely contacted with a housing made of an aluminum plate (Al-Mg-based 5052, thickness of 0.5 mm) as the cooling member 40 to construct a cooling system 200. This cooling system 200 was placed on a ceramic heater (micro-ceramic heater MS-3 manufactured by SAKAGUCHI E.H VOC CORP. heat generation: 9 W) as a heat source in such a manner that the bottom surface of the projecting portion 30b of the thermally conductive elastomer piece 30 closely contacted the ceramic heater. In this state, the ceramic heater was powered. After ten minutes, the temperature t1 of the center portion of the top surface of the ceramic heater (namely, at the interface with the projecting portion 30b) and the temperature t2 of the peripheral portion of the thermal diffusion sheet 10 were measured (distance between the measurement positions of the temperatures t1 and t2: 40 mm), the temperature t1 was 72.1°C and the temperature t2 was 29.6°C.

### (Example 2)

Firstly, 70 parts by weight of graphitized carbon fibers (manufactured by Nippon Graphite Fiber Corporation, average fiber diameter: 9µm, average fiber length: 100 µm) and 150 parts by weight of aluminum oxide powders (manufactured by Showa Denko K.K., in spherical shape, average particle size: 3.5 µm) as thermally conductive fillers were mixed with 100 parts by weight of liquid silicone rubber (manufactured by GE Toshiba Silicone Co., Ltd.) as a polymer matrix material. The resultant mixture was vacuum defoamed to prepare a thermally conductive polymer composition.

A thermal diffusion sheet 10 consisting of a graphite sheet having a thickness of 0.13 mm, a length of 30 mm, and a width of 60 mm (manufactured by GrafTech International Ltd.) was prepared. The thermal conductivity of the graphite sheet in the thickness direction was 7 W/m·K, and the thermal conductivity of the graphite sheet in the direction parallel to the surface of the sheet was 240 W/m·K. A opening 20 being 6 mm long and 6 mm wide was formed through the thermal diffusion sheet 10 at a desired position where the thermally conductive elastomer piece 30 will be incorporated thereto. This thermal diffusion sheet 10 was placed in a cavity of a predetermined mold. Subsequently, said thermally conductive polymer composition was injected into the cavity of the mold. The composition in the cavity was applied with a magnetic field (magnetic flux density of 10 tesla) in such a manner that the lines of magnetic force became substantially perpendicular to the surface of the thermal diffusion sheet 10. Thereby, the graphitized carbon fibers contained in the thermally conductive polymer composition were oriented such that the longitudinal axes of the graphitized carbon fibers were aligned substantially perpendicular to the surface of the thermal diffusion sheet 10. Then, the composition was thermally cured while the orientation of the graphitized carbon fibers was maintained. As a result, a thermally conductive member 100 shown in Fig. 3 in which a plate-like thermally conductive elastomer piece 30 (hardness of 40) so as to cover the opening 20 was obtained. The thermally conductive elastomer piece 30 had a thickness of 0.4 mm in total of the portions 30a, 30b, and 30c, and a length of 10 mm and a width of 10 mm in the portion of the projecting portions 30a, 30b.
The graphitized carbon fibers contained in the thermally conductive elastomer piece 30 were oriented in the direction substantially perpendicular to the surface of the thermal diffusion sheet 10 (in the Z direction of Fig. 3).

In the obtained thermally conductive member 100, the thermal conductivities of the thermally conductive elastomer piece 30 in the directions perpendicular to the surface of the thermal diffusion sheet 10 and parallel to the surface were 5.7 W/m·K and 2.2 W/m·K, respectively.

The top surface of the projecting portion 30a of the thermally conductive elastomer piece 30 of the obtained thermally conductive member 100 was closely contacted with a housing composed of an aluminum plate (Al-Mg-based 5052, thickness of 0.5 mm) as the cooling member 40 to construct a cooling system 200. This cooling system 200 was placed on a ceramic heater (micro-ceramic heater MS-3 manufactured by SAKAGUCHI E.H VOC CORP. heat generation: 9 W) as a heat source in such a manner that the bottom surface of the projecting portion 30b of the thermally conductive elastomer piece 30 closely contacted the ceramic heater. In this state, the ceramic heater was powered. After ten minutes, the temperature t1 of the center portion of the top surface of the ceramic heater (namely, at the interface with the projecting portion 30b) and the temperature t2 of the peripheral portion of the thermal diffusion sheet 10(interval between the measurement positions of the temperatures t1 and t2: 40 mm) were measured. The temperature t1 was 64.2°C and the temperature t2 was 35.1°C.

### (Example 3)

Firstly, 70 parts by weight of graphitized carbon fibers (manufactured by Nippon Graphite Fiber Corporation, average fiber diameter: 9µm, average fiber length: 100 µm) and 150 parts by weight of aluminum oxide powders (manufactured by Showa Denko K.K., in spherical shape, average particle size: 3.5 µm) as thermally conductive fillers were mixed with 100 parts by weight of liquid silicone rubber (manufactured by GE Toshiba Silicone Co., Ltd.) as a polymer matrix material. The resultant mixture was vacuum defoamed to prepare a thermally conductive polymer composition.

A thermal diffusion sheet 10 consisting of a graphite sheet having a thickness of 0.13 mm, a length of 30 mm, and a width of 60 mm (manufactured by GrafTech International Ltd.) and an aluminum foil having a thickness of 0.015 mm laminated on the both sides of the graphite sheet was provided. The thermal conductivity of the graphite sheet in the thickness direction was 7 W/m·K, and the thermal conductivity of the graphite sheet in the direction parallel to the surface of the sheet was 240 W/m·K. An opening 20 being 6 mm long and 6 mm wide was formed through the thermal diffusion sheet 10 at a desired position where the thermally conductive elastomer piece 30 will be incorporated thereto. The thermal diffusion sheet 10 was placed in the cavity of a predetermined mold. Subsequently, said thermally conductive polymer composition was injected into the cavity of the mold. The composition in the cavity was then applied with a magnetic field (magnetic flux density of 10 tesla) in such a manner that the lines of magnetic force extend substantially perpendicular to the surface of the thermal diffusion sheet 10. Thereby, the graphitized carbon fibers contained in the thermally conductive polymer composition were oriented in a direction substantially perpendicular to the surface of the thermal diffusion sheet 10. Then, the composition was thermally cured while the orientation of the graphitized carbon fibers was maintained. As a result, a thermally conductive member 100 shown in Fig. 3 in which a plate-like thermally conductive elastomer piece 30 (hardness of 40) was integrated with the thermal diffusion sheet 10 to so as cover the opening 20 was obtained. The thermally conductive elastomer piece 30 had a thickness of 0.4 mm in total of the portions 30a, 30b, and 30c, and a length of 10 mm and a width of 10 mm in the portion of the projecting portions 30a, 30b.

The graphitized carbon fibers contained in the thermally conductive elastomer piece 30 of the obtained thermally conductive member were aligned in the direction (in the Z direction of Fig. 3) perpendicular to the surface of the thermal diffusion sheet 10. In the obtained thermally conductive member, the thermal conductivities of the thermally conductive elastomer piece 30 in the direction perpendicular to the surface of the thermal diffusion sheet 10 and the direction parallel to the said surface were measured were 5.7 W/m·K and 2.2 W/m·K, respectively.

Next, the top surface of the projecting portion 30a of the thermally conductive elastomer piece 30 of the obtained thermally conductive member 100 was contacted with a housing made of an aluminum plate (Al-Mg-based 5052, thickness of 0.5 mm) as the cooling member 40 to construct a cooling system 200. This cooling system 200 was placed on a ceramic heater (micro-ceramic heater MS-3 manufactured by SAKAGUCHI E.H VOC CORP. heat generation: 9 W) as a heat source in such a manner that the bottom surface of the projecting portion 30b of the thermally conductive elastomer piece 30 closely contacted the ceramic heater. In this state, the ceramic heater was powered. After ten minutes, the temperature t1 of the center portion of the top surface of the ceramic heater (namely, at the interface with the projecting portion 30b) and the temperature t2 of the peripheral portion of the thermal diffusion sheet 10 (interval between the measurement positions of the temperatures t1 and t2: 40 mm) after 10 minutes, the temperature t1 was 60.9°C and the temperature t2 was 38.8°C.

### (Example 4)

A thermal diffusion sheet 10 consisting of a graphite sheet having a thickness of 0.13 mm, a length of 30 mm, and a width of 60 mm (manufactured by GrafTech International Ltd.) was provided. The thermal conductivity of the graphite sheet in the thickness direction was 7 W/m·K, and thermal conductivity of the graphite sheet in the direction parallel to the surface of the sheet was 240 W/m·K. An opening 20 being 6 mm long and 6 mm wide was formed through thermal diffusion sheet 10 at a desired position where the thermally conductive elastomer piece 30 will be incorporated thereto.

Meanwhile, 70 parts by weight of graphitized carbon fibers (manufactured by Nippon Graphite Fiber Corporation, average fiber diameter: 9µm, average fiber length: 100 µm) and 150 parts by weight of aluminum oxide powders (manufactured by Showa Denko K.K., in spherical shape, average particle size: 3.5 µm) were mixed with 100 parts by weight of liquid silicone rubber (manufactured by GE Toshiba Silicone Co., Ltd.). The resultant mixture was vacuum defoamed to prepare a thermally conductive polymer composition. Subsequently, the said thermally conductive polymer composition was injected into a cavity of a mold corresponding to a desired plate form. The thermally conductive polymer composition in the cavity was applied with a magnetic field (magnetic flux density of 10 tesla) in such a manner that the lines of magnetic force extended the thickness direction of the plate. Thereby, the graphitized carbon fibers contained in the thermally conductive polymer composition were oriented in the thickness direction of the plate. Then the composition was thermally cured, while the orientation of the graphitized carbon fibers was maintained. As a result, a thermally conductive elastomer piece 30 (hardness of 40) in a plate having a thickness of 0.4 mm, a length of 10 mm, a width of 10 mm, and a continuous slit formed on the outer periphery sides surrounding a base portion 30c was obtained. This thermally conductive elastomer piece 30 was assembled with the thermal diffusion sheet 10 through the opening 20 to obtain a thermally conductive member 100 shown in Fig. 3.

The graphitized carbon fibers contained in the thermally conductive elastomer piece 30 of the obtained thermally conductive member were oriented in the direction (in the Z direction of Fig. 3) perpendicular to the surface of the thermal diffusion sheet 10.

In the obtained thermally conductive member, the thermal conductivities of the thermally conductive elastomer piece 30 in the direction perpendicular to the surface of the thermal diffusion sheet 10 and the direction parallel to the said surface were measured were 5.7 W/m·K and 2.2 W/m·K, respectively.

As for the thermally conductive member obtained in Example 4, the graphite sheet used, the composition of the thermally conductive elastomer, and the orientation conditions of the thermally conductive filler were identical to those in Example 2, though the manufacturing method of Example 4 differed from that of Example 2. Accordingly, the thermally conductive member obtained in Example 4 has the same characteristic properties as the thermally conductive member obtained in Example 2. Therefore, the heat radiation evaluation was omitted.

### (Comparative Example 1)

A thermal diffusion sheet 7, which was a graphite sheet having a thickness of 0.13 mm, a length of 30 mm, and a width of 60 mm (manufactured by GrafTech International Ltd.) was applied with an acrylic resin-based pressure sensitive adhesive in a thickness of 5 µm, and placed on a ceramic heater (micro-ceramic heater MS-3 manufactured by SAKAGUCHI E.H VOC CORP. heat generation: 9 W) as a heat source. The thermal conductivity in the thickness direction of the graphite sheet was 7 W/m·K, and the thermal conductivity of the graphite sheet in the direction parallel to the surface was 240 W/m·K. The top surface of the thermal diffusion sheet 7 was directly contacted to a housing made of an aluminum plate (Al-Mg-based 5052, thickness of 0.5 mm) as a cooling member to construct a cooling system. In this case, the thermally conductive elastomer of Example 1 was not used.

In this state, the ceramic heater was powered. After ten minutes, the temperature t1 of the center portion of the top surface of the ceramic heater (namely, at the interface with the projecting portion 30b) and the temperature t2 of the peripheral portion of the thermal diffusion sheet 10 (interval between the measurement positions of the temperatures t1 and t2: 40 mm) were measured. The temperature t1 was 88.0°C and the temperature t2 was 25.5°C. These results show that the heat from the ceramic heater as the heat source was not successfully conducted and diffused to the thermal diffusion sheet, because of low thermal conduction efficiency due to poor contact between the ceramic heater and the thermal diffusion sheet and due to the fact that the heat from the ceramic heater was conducted to the thermal diffusion sheet through the surface of the thermal diffusion sheet.

The present examples and embodiments are to be considered as illustrative and not restrictive and the invention is not to be limited to the details given herein, but may be modified within the scope and equivalence of the appended claims.

Attention is directed to all papers and documents which are filed concurrently with or previous to this specification in connection with this application and which are open to public inspection with this specification, and the contents of all such papers and documents are incorporated herein by reference.

All of the features disclosed in this specification (including any accompanying claims, abstract and drawings), and/or all of the steps of any method or process so disclosed, may be combined in any combination, except combinations where at least some of such features and/or steps are mutually exclusive.

Each feature disclosed in this specification (including any accompanying claims, abstract and drawings) may be replaced by alternative features serving the same, equivalent or similar purpose, unless expressly stated otherwise. Thus, unless expressly stated otherwise, each feature disclosed is one example only of a generic series of equivalent or similar features.

The invention is not restricted to the details of the foregoing embodiment(s). The invention extends to any novel one, or any novel combination, of the features disclosed in this specification (including any accompanying claims, abstract and drawings), or to any novel one, or any novel combination, of the steps of any method or process so disclosed.

## Claims

1. A thermally conductive member (100) **characterized by:**
a thermal diffusion sheet (10) having at least one opening (20) with an inner periphery; and
a thermally conductive elastomer piece (30) passing through the opening (20) of the sheet, wherein the thermally conductive elastomer piece (30) includes at least one base portion (30c) fitting with the inner periphery of the opening (20) of the thermal diffusion sheet (10) and at least one projecting portion (30a, 30b) connected to the base portion (30c) and projecting out from the surface of the thermal diffusion sheet (10).

2. The thermally conductive member (100) according to claim 1 **characterized in that**: the projecting portion (30a, 30b) of the thermally conductive elastomer piece (30) is larger than the opening (20) of the thermal diffusion sheet (10) in cross-section.

3. The thermally conductive member (100) according to claims 1 or 2 **characterized in that**: the thermal conductivity of the thermal diffusion sheet (10) in a direction parallel to the surface of the sheet is higher than thermal conductivity of the thermal diffusion sheet (10) in a thickness direction of the sheet.

4. The thermally conductive member (100) according to any one of preceding claims **characterized in that**: the thermal diffusion sheet (10) is one of a graphite sheet and a composite sheet including a graphite sheet and an aluminum foil provided on the graphite sheet.

5. The thermally conductive member (100) according to any one of preceding claims **characterized in that**: the thermally conductive elastomer piece (30) has electrically insulative properties.

6. The thermally conductive member (100) according to any one of preceding claims **characterized in that**: the thermally conductive elastomer piece (30) contains at least one type of thermally conductive filler selected from carbon fibers, carbon nanotubes, metal nitrides, metal oxides, metal carbides, and metal hydroxides.

7. The thermally conductive member (100) according to claim 6 **characterized in that**: the thermally conductive filler in the thermally conductive elastomer piece (30) is oriented in a specific direction, and thereby thermal conductivity of the thermally conductive elastomer piece (30) in a direction substantially perpendicular to the surface of the thermal diffusion sheet (10) is higher than thermal conductivity in a direction parallel to the surface of the thermal diffusion sheet (10).

8. A cooling system **characterized by**:
a thermal diffusion sheet (10) having at least one opening (20) with an inner periphery;
a thermally conductive elastomer piece (30) passing through the opening (20) of the sheet, wherein the thermally conductive elastomer piece (30) includes at least one base portion (30c) fitting with an inner periphery of the opening (20) of the thermal diffusion sheet (10) and at least one projecting portion (30a, 30b) connected to the base portion (30c) and projecting out from the surface of the thermal diffusion sheet (10); and
a cooling member (40) in close contact with the projecting portion (30a, 30b) of the thermally conductive elastomer piece (30).

9. The cooling system according to claim 8 **characterized in that**: the cooling system is for mounting on an apparatus and the cooling member (40) is a housing for the apparatus.

10. A method for producing a thermally conductive member (100) including a thermal diffusion sheet (10) having at least one opening (20) with an inner periphery, and a thermally conductive elastomer piece (30) passing through the opening (20) of the sheet, wherein the thermally conductive elastomer piece (30) includes at least one base portion (30c) fitting with the inner periphery of the opening (20) and at least one projecting portion (30a, 30b) connected to the base portion (30c) and projecting out from the surface of the thermal diffusion sheet (10), **characterized by:**
forming the at least one opening (20) through the thermal diffusion sheet (10);
preparing a composition containing a polymer matrix and a thermally conductive filler; and
insert molding the composition with the thermal diffusion sheet (10) to produce the thermally conductive elastomer piece (30) passing through the opening (20) of the thermal diffusion sheet (10).

11. The method according to claim 10 **characterized in that**:
during the insert molding, the thermally conductive filler in the composition is oriented in a specific direction by application of a magnetic field.

12. A method for producing a thermally conductive member (100) including a thermal diffusion sheet (10) having at least one opening (20) with an inner periphery, and a thermally conductive elastomer piece (30) passing through the opening (20) of the sheet, wherein the thermally conductive elastomer piece (30) includes at least one base portion (30c) fitting with the inner periphery of the opening (20) and at least one projecting portion (30a, 30b) connected to the base portion (30c) and projecting out from the surface of the thermal diffusion sheet (10), **characterized by:**
forming the at least one opening (20) through the thermal diffusion sheet (10);
preparing a composition containing a polymer matrix and a thermally conductive filler; and
molding the composition into the thermally conductive elastomer piece (30) in a predetermined shape;
assembling the thermally conductive elastomer piece (30) with the thermal diffusion sheet (10) through the opening (20) of the sheet.

13. The method according to claim 12 **characterized in that**:
during the molding, the thermally conductive filler in the composition is oriented in a specific direction by application of a magnetic field.
